# EUROPEAN PATENT APPLICATION

(11) **EP 1 088 540 A1**
(43) Date of publication of application: **04.04.2001**
(21) Application number: 00308128.8
(22) Date of filing: 18.09.2000
(51) Int. Cl.: A61K 7/00, A61K 7/48

(54) **Method for forming a silicone coating on a substrate**

(30) Priority: 29.09.1999 EP 99402378
(71) Applicant: Dow Corning France, 69003 Lyon (FR)
(72) Inventor: Aguadisch, Louis Michel Jacques, 06560 Valbonne (FR); Fonta, Frederique, 06600 Antibes (FR); Mallard, Claire, 06250 Mougins le Haut (FR)
(74) Representative: Vandamme, Luc Johan Roger

(57) **Abstract**

A method of forming a silicone coating on a substrate by depositing a water in silicone oil emulsion comprising 1 to 10 wt. % of a surfactant, 9 to 65 wt % of a volatile silicone fluid and 28 to 90 wt % of an aqueous phase onto a substrate. Shear stress is then applied to the composition to distribute the composition on the substrate. As the shear stress is applied, the emulsion breaks and inverts to a silicone coating on the substrate. This method provides a novel method of delivering a silicone coating on a substrate. It also provides a delivery vehicle for a variety of water soluble and oil soluble actives.

## Description

The present invention relates to a method of forming a silicone coating on a substrate from a water in silicone oil emulsion which breaks and inverts under the stress of application.

Water-in-silicone oil emulsions are known in the art. Typically, such emulsions comprise water dispersed in silicone oils stabilized with surfactants and/or other additives. It is likewise know in the art to use these emulsions for depositing silicone on substrates such as skin and/or hair.

Examples of patents which describe such emulsions include U.S. Patent Number 3,489,690 which describes water-in-silicone oil emulsions using certain polyoxyalkylene alcohols as the emulsifiers. Similarly, US Patent Numbers 4,122,029 and 4,311,695 teach the formation of such emulsions by incorporating water-in-oil surfactants having an HLB value of from 2 to 10, certain polydiorganosiloxane-polyoxyalkylene copolymers and other adjuctants. Likewise, Canadian Patent No. 742,289 teaches water in silicone oil emulsions in which the oil phase is a siloxane-polyoxyalkylene copolymer.

Each of the above references teach emulsions which are stable and useful for applying active ingredients to the skin. They do not, however, teach the emulsions herein that break in the process of delivery and, as such, cannot be deposited by the process of this invention.

It is one of the objects of the invention to provide a process for delivering a silicone coating on a substrate from a water in silicone oil emulsion which phase inverts during application. It is another object to provide a process for delivering an active ingredient to a substrate from a water in silicone oil emulsion which phase inverts during application.

The present invention claims a method of forming a silicone coating on a substrate comprising depositing a composition comprising a water in silicone oil emulsion onto the substrate and then applying shear stress to the composition to insure distribution of the composition on the substrate, the improvement comprising the use of an emulsion comprising 1 to 10 wt. % of a surfactant, 9 to 65 wt % of a volatile silicone fluid and 28 to 90 wt % of an aqueous phase, wherein the emulsion is one which breaks when the shear stress is applied to deposit a silicone coating on the substrate.

These emulsions can act as delivery vehicles for active ingredients in either the aqueous or the oil phase.

This invention provides a water in silicone oil emulsion that can be used for delivering silicone coatings and/or active ingredients to a substrate. These emulsions are applied onto substrates and then shear is applied, generally by rubbing, to spread it on the substrate. As the shear is applied, the emulsion starts to increase its viscosity (i.e., the applicator feels an increase in resistance during application). At some stage in the application of shear, the emulsion rapidly and distinctly breaks and inversion occurs, i.e., the emulsion converts from a water in oil emulsion to a silicone oil film with the aqueous phase expelled from the emulsion and deposited on its surface. As this occurs, the applicator senses a distinct drop in resistance (viscosity), i.e., a radical 'slipping' sensation is readily apparent.

Therefore, as used herein, the description of emulsions which 'break', 'phase invert' or 'invert' is meant to encompass emulsions which go through this phase transformation as shear such as, for example, the shear associated with application of the emulsion on the substrate, is applied to the emulsion. This can be readily ascertained, for example, by depositing the emulsion on a substrate and using an implement such as, for example, a finger or a plastic applicator, to rub the emulsion back and forth onto the substrate several times and observe for the desired transformation behavior.

We have also discovered that by modifying the formulation of the emulsion, one can vary the properties that the emulsion displays on application. For instance, one can vary the viscosity increase during the early application phase, the magnitude of the 'slipping' sensation achieved at the end of the deposition process and the amount of aqueous phase expelled from the emulsion.

By the above process, one can deposit a silicone film onto a substrate. This process is especially advantageous in that the substrate onto which the silicone oil is deposited typically doesn't have an oily feel after application. Moreover, the object used to induce the shear during application (e.g., fingers) typically doesn't have an oily residue on its surface, e.g., no oily film on the fingers following a deposition onto a substrate.

In addition, it should be noted that the above properties of the emulsion provide a clear signal to the applicator to indicate when the silicone film has been deposited. Specifically, as the applicator induces the above described shear stress during application of the emulsion onto a substrate, the rapid change from a viscous feeling substance to a 'slippery' sensation provides a tactile indication to the user that the silicone film has been deposited and the rubbing can stop, if desired.

The present invention, therefore, provides a process of depositing a silicone coating on a substrate from a water in silicone oil emulsion which is able to phase invert during application. The silicone oil phase forms a film that adheres to the substrate to be treated and the aqueous phase migrates out of the emulsion and onto the surface of the silicone where it can evaporate. If the application is by rubbing with fingers, this process leaves a hydrated layer as the main feel. As such, the process provides both the benefits of an oil in water emulsion (e.g., no oily feel, ease of application, compliance, etc.) and the benefits of a water in oil emulsion (e.g., film protection, substantivity, water repellency, emolliency, etc.). These emulsions, therefore, have utilities in topical pharmaceutical formulations, personal care applications, household care applications, veterinary applications and other industries involved in coating substrates with silicone films such as fiber, textile, paper and the like. It should be noted that while rubbing with fingers is an example of application methods, other means such as mechanical spreaders, rollers and the like are functional herein.

The water in oil silicone emulsions used in the present invention generally comprises an oily phase derived from a volatile silicone fluid; an aqueous phase; and a surface active agent (surfactant). Ingredients which may optionally be incorporated in the emulsions include those that increase the viscosity of the oil; those that can modulate the inversion process; and active ingredients which can be incorporated into either or both of the aqueous or oil phase. These optional ingredients can include, for example, silicone gums, silicone oils such as dimethiconol blends, silicone waxes, and a variety of the well known active ingredients used in healthcare, personal care, home care, or other similar arts.

Generally, the ingredients of the present invention will be used in the following amounts:

| | |
|---|---|
| Surfactant | 1 to 10 wt. % |
| Volatile Silicone | 9 to 65 wt% |
| Aqueous phase | 28 to 90 wt% |
| Silicone gums | 0 to 7 wt% |
| Silicone oils | 0 to 10 wt% |
| Silicone wax | 0 to 5 wt% |
| Active ingredients | 0 to 30 wt% |

We have also discovered that the viscosity increase during application can be increased proportionally by increasing the amount of surfactant; increasing the amount of silicone gum; increasing the volatility of the silicone; and increasing the viscosity of the silicone by combining it with, for example, the silicone wax. Likewise, the aqueous phase expulsion can be increased by increasing the amount of aqueous phase in the emulsion.

On the contrary, we have discovered that some excipients inhibit the phase inversion effect. For instance, there is a reduction in inversion (i.e., less water expulsion during phase inversion) when silicone wax in an amount above that indicated above; when pharmaceutical excipients which present a fatty or greasy feel like polyglycols, fatty esters, fatty acids, mineral oils, silicone oil, etc. are added to the emulsion; and when low volatility pharmaceutical solvents such as ethanol are added to the emulsion.

The volatile silicone fluids (siloxanes or polysiloxanes) which can be used in the present invention are not critical and generally any which have a viscosity of up to about 50 millipascal-seconds at 25° C such that they volatilize in the ambient environment can be used herein.

Generally, the volatile silicone fluids correspond to the average unit formula (R)ₐSiO_{(4-a)/2} where R is H, an alkyl of 1-6 carbon atoms or an aryl *a* has an average value of from 2 to 3. Preferably, R is methyl. Such preferable fluids comprises siloxane units joined by Si-O-Si bonds selected from the group consisting of (CH₃)₃SiO_{1/2}, (CH₃)₂SiO_{2/2}, CH₃SiO_{3/2} and SiO_{4/2} units taken in such molar amounts so that there is an average of from approximately two to three methyl groups per silicon in the fluid and said fluid has a viscosity of no more than 50 millipascal-seconds at 25° C.

More preferably, the volatile silicone fluid consists essentially of dimethylsiloxane units, and optionally, trimethylsiloxane units. Of particular interest in the present invention are methylsiloxane fluids having a viscosity of less than 10 millipascal-seconds at 25°C such as the cyclopolysiloxanes of the general formula {(CH₃)₂SiO}ₓ and linear siloxanes of the general formula (CH₃)₃SiO{(CH₃)₂SiO}_{y}Si(CH₃)₃ wherein x is an integer of from 3 to 8 and *y* is an integer of from 0 to 4.

Preferred volatile silicone fluids include cyclic silicones such as hexamethylcyclotrisiloxane, octamethylcyclotetrasiloxane, decamethylcyclopentasiloxane, and the like, linear silicones such as hexamethyldisiloxane, octamethyltrisiloxane, decamethyltetrasiloxane, and the like and mixtures of any of the above volatile silicone fluids. The preferred volatile silicone fluid is hexamethyldisiloxane.

These volatile silicone fluids and methods for their manufacture are known in the art and many are commercially available.

The emulsion of the invention will normally comprise from 9 to 65 %, preferably from 10 to 50% by weight of the volatile silicone fluid.

The surfactants to be used in the present invention include organic surfactants, silicone surfactants and mixtures thereof.

Examples of silicone surfactants include polydiorganosiloxane-polyoxyalkylene block copolymers containing at least one polydiorganosiloxane block and at least one polyoxyalkylene block. The polyoxyalkylene blocks may be bonded to the polydiorganosiloxane blocks with silicon-oxygen-carbon bonds and/or with silicon-carbon bonds, although it is preferred that the block copolymer have silicon-carbon bonding instead of the more hydrolyzable silicon-oxygen-carbon bonding joining the polyoxyalkylene blocks to the polydiorganosiloxane blocks.

The polydiorganosiloxane blocks of the silicone surfactants herein comprise siloxane units which are interlinked by Si-O-Si linkages and which have the formula R'_{b}SiO_{(4-b)/2}. The value of b may range from 0 to 3, with the provision that there is an average of approximately 2, e.g., from 1.9 to 2.1 R' groups for every silicon in the block copolymer. Suitable siloxane units thus include R'₃SiO_{1/2}, R'₂SiO_{2/2}, R'SiO_{3/2}, and SiO_{4/2} siloxane units taken in such molar amounts so that b has an average value of approximately 2. The siloxanes may be linear, cyclic and/or branched.

The R' radicals of the above silicone surfactant may be any radical selected from the group consisting of monovalent hydrocarbon groups of 1-20 carbon atoms such as methyl, ethyl, vinyl, phenyl, and a divalent radical bonding a polyoxyalkylene block to the polydiorganosiloxane block. It is preferred that at least 95 percent of all R radicals are methyl radicals and preferably there is at least one methyl radical bonded to each silicon atom.

Divalent R' radicals bonding a polyoxyalkylene block to a polydiorganosiloxane block may be bonded to the siloxane units of said polydiorganosiloxane block by a silicon-oxygen bond or by a silicon-carbon bond. As noted above, silicon-carbon bonding between polydiorganosiloxane blocks and polyoxyalkylene blocks is preferred. These divalent R' radicals preferably contain no more than 6 carbon atoms. Illustrative of divalent R' radicals include -O-, -CₘH₂ₘO-, -CₘH₂ₘ- and -CₘH₂ₘCO₂- where m is an integer, preferably 1-8 and most preferably 2-6.

Examples of the siloxane units include R'₃SiO_{1/2} units such as Me₃SiO_{1/2}, Me₂(CH₂CH)SiO_{1/2}, Me₂(C₆H₅)SiO_{1/2}, Me(C₆H₅)(CH₂CH)SiO_{1/2}, Me₂(CH₃CH₂)SiO_{1/2}, Me₂QSiO_{1/2}, MeQ₂SiO_{1/2}, Q₃SiO_{1/2}, Q₂(CH₃CH₂)SiO_{1/2}, and Me(C₆H₅)(Q)SiO_{1/2}; R'₂SiO_{2/2} units such as Me₂SiO_{2/2}, Me(C₆H₅)SiO_{2/2}, Me(CH₂CH)SiO_{2/2}, (C₆H₅)₂SiO_{2/2}, MeQSiO_{2/2}, and Q(C₆H₅)SiO_{2/2}; R'SiO_{3/2} units such as MeSiO_{3/2}, C₆H₅SiO_{3/2}, CH₂CHSiO_{3/2}, CH₃CH₂SiO_{3/2} and QSiO_{3/2} and SiO_{4/2} units. Me denotes methyl and Q denotes a divalent R' radical bonding a polyoxyalkylene block to the polydiorganosiloxane block.

It is to be understood that the siloxane surfactant may comprise one or more of said polydiorganosiloxane blocks. The number of and average molecular weight of the polydiorganosiloxane blocks in the copolymer is related to the desired weight ratio, hereinafter described, of said blocks in the copolymer. Preferably, the silicone surfactant comprises one polydiorganosiloxane block having bonded thereto one or more polyoxyalkylene blocks.

Examples of the polyoxyalkylene blocks comprise oxyethylene units of the formula -CH₂CH₂O-, oxypropylene units of the formula -CH₂CH(CH₃)O-, or mixtures thereof. Preferably, an average of at least half of the oxyalkylene units in the polyoxyalkylene blocks are oxyethylene units. The polyoxyalkylene blocks thus correspond to the formula {-CH₂CH₂O-}ₚ{-CH₂CH(CR₃)O-}_{q} wherein the oxyalkylene units may be arranged in any suitable fashion such as random, alternating and block.

The polyoxyalkylene blocks are bonded to the polydiorganosiloxane blocks of said copolymer by at least one terminal portion of said polyoxyalkylene block, said bonding being by way of a divalent R' radical, hereinbefore described. It is to be understood that said bonding may be by both terminal portions of said polyoxyalkylene block in those block copolymers comprising more than one polydiorganosiloxane block. Any terminal portion of the polyoxyalkylene block that is not bonded to a polydiorganosiloxane block is satisfied by a terminating radical. The type of said terminating radical is not critical and may be monovalent, thereby terminating one polyoxyalkylene block, or polyvalent, thereby terminating more than one polyoxyalkylene block. Said terminating radicals are made up of atoms selected from the group consisting of carbon, hydrogen, nitrogen, and oxygen. Illustrative of said terminating radical are hydrogen; hydroxyl; alkyl, such as methyl, ethyl, propyl, butyl; benzyl; aryl, such as phenyl; alkoxy such as methoxy, ethoxy, propoxy, butoxy; glyceroxy; benzyloxy; aryloxy, such as phenoxy; alkenyloxy, such as vinyloxy and allyloxy; acyloxy, such as acetoxy, acryloxy and propionoxy and amino such as dimethylamino.

The polydiorganosiloxane-polyoxyalkylene block copolymers useful in the emulsion compositions of this invention may be prepared by any method suitable for block copolymer formation. Many of these methods are known in the art and are described in, for example, US Patent Number 4,122,029. Additionally, many such block copolymers are commercially available.

As note above, other surfactants can also be used in the present invention. These include, for example, non-ionic, anionic and cationic organic surfactants such as sorbitan monooleate.

The emulsion according to the invention will normally comprise from 0.1 to 20%, preferably from 1 to 10% by weight of the surfactant. As noted, other conventional surfactants may be added as co-surfactants.

The emulsion also comprises an aqueous phase comprising water and any other ingredients which may be desirably added to this phase as the disperse phase. Such optional ingredients include, for example, moisturizers such as glycerin, sorbitol, urea, lactic acid, propylene glycol, polyethylene glycols and copolmers, glucose derivatives and the like, emollients, humectants, thickening agents such as cellulose derivatives, polyvinyl alcohol, polyacrylates, alginates, gums, starch, polyvinyl pyrrolidone, and the like, preservatives such as parabens and the like, phenols such as chlorhexidine, and the like, antioxidants such as ascorbic acid and esters, thiourea, sodium bisulfite, tocopherols, and the like, chelating agents such as EDTA, citric acid, and the like, flavoring agents, dyes, buffering agents, solubilizing agents such as benzalkonium chloride, cyclodextrins, lecithins, poloxamers, polysorbates, sodium lauryl sulfate, sorbitan fatty esters, glycerol fatty acid esters, polyoxyethylene and derivatives, and the like, penetration enhancers, alcohols such as ethanol, isopropanol, benzyl alcohol, and the like, glycols, mono and polysaccharides, colloidal solids, quaternary amines such as benzalkonium chloride, and the like, and acids such as sorbic acid, benzoic acid, salicyclic acid, and the like.

The amount of the aqueous phase may lie in the range not exceeding 90% by weight and amounts in the range of 28 to 90 wt. % are preferred.

The emulsions of the invention can also optionally contain silicone gums.
While such gums are typically high molecular weight polydimethylsiloxanes terminated with unreactive groups such as trimethylsilyl or reactive groups such as dimethylhydroxysilyl or dimethylvinylsilyl, nearly any silicone gum, or mixtures thereof, will function herein. Most preferably, the silicone gum is a hydroxyl-terminated polydimethylsiloxane.

Silicone gums typically have viscosities up to 50 million mm²/s at 25°C and have number average molecular weights (Mn) of up to 700,000 or more. Preferably, the gums have an Mn of about 200,000 to 400,000.

Such gums and methods for their production are known in the art as exemplified by Noll, Chemistry and Technology of Silicones, Academic Press, 1968. In addition, silicone gums are commercially available from, for example, Dow Corning Corporation.

Generally, the silicone gums are added in an amount of 0 to 7 wt %, preferably 0.1 to 5 wt %.

The emulsions of the invention can also optionally contain silicone waxes. Nearly any silicone wax, or mixtures thereof, will function herein.

Preferred silicone waxes suitable for use in the present invention include alkylmethylsiloxane copolymers having the following formulations such as
1. (RMeSiO)ₐ(Me₂SiO)_{b}
   or
2. R'Me₂(RMeSiO)_{y} (Me₂SiO)_{z} SiMe₂R'
wherein R is CₙH_{2n+ 1}, R' is R or Me, Me is CH₃, n is 5 to 45, preferably 10-30, a is an integer from 3 to 10, b is an integer of 0 to 10, a + b is 3 to 10 and y and z are independently 0 or a positive integer, provided either a or y are sufficient to result in a waxy material and/or R' is R and the resultant material is waxy in character. Typically, the silicone waxes of the present invention have melting points of between about 30° C and about 100°C.

Preferably, the silicone wax comprises a trimethylsiloxy-terminated dimethyl, methyloctadecylsiloxane.

Methods for the preparation of such materials are known in the art, and such methods are described in, for example, U.S. Pat. No. 5,017,221 which issued May 21, 1991, and U.S. Pat. No. 5,160,494 which issued Nov. 3, 1992, both of which are incorporated herein by reference. Basically, such methods involve the reaction of a linear siloxane having SiH functionality in the chain with a cyclic siloxane containing Me₂SiO units, and contacting the reaction product with a slight stoichiometric excess of an alkene in the presence of a platinum on carbon catalyst.

Generally, the waxes are added in an amount of 0 to 5 wt %, preferably 0.1 to 4 wt. % and are typically added to formulations of the invention after being melted or dissolved in a solvent.

The emulsions of the invention can also optionally contain silicone fluids. These fluids can be polydimethylsiloxanes terminated with unreactive groups such as trimethylsilyl or reactive groups such as dimethylhydroxysilyl or dimethylvinylsilyl. Preferably, the silicone fluid is a trimethylsilyl or dimethylhydroxysilyl end-capped polydimethylsiloxane and more preferably dimethiconol blends.

Silicone fluids can have a wide range of viscosities such as from about 20 up to about 150,000 mm²/s at 25°C and preferably from about 50 up to about 100,000 mm²/s at 25°C.

Such fluids and methods for their production are known in the art as exemplified by Noll, Chemistry and Technology of Silicones, Academic Press, 1968. In addition, silicone fluids are commercially available from, for example, Dow Corning Corporation.

Generally, the silicone fluids are added in an amount of 0 to 20 wt %, preferably 1 to 10 wt %.

The water-in-oil emulsion according to the invention can act as a delivery vehicle to provide a matrix to serve as a vehicle in which active ingredients can be dissolved, dispersed or suspended. Examples of actives ingredients include personal care actives, pharmaceutical actives, and others including, for example, hair growth promoters, moisturizers such as urea, sodium hyaluronate, sodium pyrrolidonecarboxylate, sodium lactate, natural moisturising factor substances, and the like, softening products such as D-panthenol and the like, emollients such as polyethylene glycol, emollient oils (mineral, vegetable, synthetic), and the like, humectants such as glycerol, propylene glycol, sorbitol, butylene glycol, and the like, restructuring products such as allantoin, and the like, astringents such as antiperspirants including organic and inorganic salts of metals such as aluminium, zirconium, zinc and mixture thereof, UV protection products such as 2-phenylbenzadimidazole-5-sulphonic acid, and the like, UV absorbers such as octyl methoxy cinnamate, butyl methoxy dibenzoil methane, ethoxylated p-aminobenzoic acid ester and benzophenone, and the like, sunscreen such as microfine titanium dioxide, and the like, refreshing products such as menthol, and the like, nourishing products such as acacia honey, oil and water soluble vitamins, trace elements or dyes and the like, deodorants, anti-acne agents, conditioners, skin lighteners such as magnesium ascorbyl phosphate, herbal extracts, bleaches, whitening ( hydroquilone, Mg ascorbyl phosphate ) and the like, thickening agents such as polyacrylates, alginates, gums, cellulosic derivatives, starch, polyvinyl alcohol and the like, antioxidants, dyes, fragrance, cleaning agents, perfumes, pearlescent aids, buffers, preservatives, antidruff agents such as zinc pyrithione and the like, anti wrinkle agents such as fruit acids, flavonoids and the like, anti aging agents, calming actives such as camomile extracts, betain, and the like, cooling agents such as menthol, ginger extracts, and the like, firming agents such as hydrocotyl extract, coneflower, bisabolol, and the like, slimming agents, pharmaceuticals such as antifungals, antibacterials, antiinflammatories (SAID and NSAID), anesthetics such as butamben picrate, lidocaine hydrochloride, xylocaine and the like, analgesics such as benzocain, dyclonine hydrochloride, and the like, antiparasitics such as lindane, agents for the treatment of calluses, corns and warts, antiseptics, keratolytics, liporegulators, antihistaminics, antivirals , antiacne, anti pruritus, enzymes, proteins, peptides, growth factors, promotors, healing factors, anti psoriasis, anticancer, hormones, antiseborrhea agents, smoking cessation agents, anti vomiting/nausea agents, cardiovascular agents, antiangina agents, antihypertension agents, central nervous system active agents, anti migraine agents, sedatives, and tranquillizers as well as related materials for, for example, treating various substrates including polishing waxes such as animal waxes including bees wax, spermaceti, and lanolin, vegetable waxes including carnauba and candellilla, mineral waxes including paraffin and petrolatum, and synthetic waxes including ethylenic polymers, polyol ether-esters, and chlorinated naphtalenes, abrasives agents such as aluminium silicates, diatomateous earth, bentonites, colloidal clay, and metallic oxides, detergents, colorants, odorants, corrosion inhibitors, fluoro polymers, and the like.

The emulsion of the invention generally comprises from 0 to 30% by weight of any active ingredient as exemplified above.

The emulsion of the invention can optionally comprise other acceptable vehicles, in addition to the aqueous phase to act as a diluent, dispersant or carrier for other materials present in the emulsion, so as to facilitate their distribution when the emulsion is applied to the substrate. Vehicles other than water can include liquid or solid solvents, propellants, and powders.

Adjuncts can generally form up to 50 % by weight of the emulsion and can conveniently form the balance of the emulsion.

The compositions of this invention may be prepared by mixing the individual components in any suitable manner in the desired proportions. Preferably the aqueous phase and the oil phase are mixed in separate containers followed by mixing said phases in conventional mixing devices for emulsions.

The compositions of the invention are useful for forming coatings on a variety of substrates including animals, including humans, fiber, textile, paper and personal and household care objects. These compositions can be used to deliver cosmetic agents, pharmaceuticals, polishes and other personal or household care materials.

The following Examples are provided so that those skilled in the art may better understand how the present invention can be practiced. They are given by way of illustration only and are not to be interpreted as limitations. All percentages and parts are by weight.

### EXAMPLES

The following examples demonstrate a variety of emulsions according to the invention and several comparative emulsions. Each emulsion was made by first mixing the oil phase and aqueous phase in separate containers. The oil phase contains the volatile silicone fluid, (HMDS = hexamethyldisiloxane, 1 cSt = octamethyltrisiloxane, D4 = octamethylcyclotetrasiloxane, and D5 = decamethylcyclopentasiloxane) the surfactant (Laurylmethicone copolyol or Sorbitan monooleate) and any silicone gum (hydroxy endblocked polydimethylsiloxane gum), dimethiconol blend (7% hydroxy endblocked polydimethylsiloxane in 93% polydimethylsiloxane 20 cst), and/or silicone wax (trimethylstearlyoxysilane) either melted or in ethanol. In addition, the oil phase contains other lipophilic excipients such as linear polydimethylsiloxanes (pdms) and mineral oil (liquid paraffin). The aqueous phase contains the water and any water soluble excipients (ethanol and propylene glycol). The emulsion is then made by slowly adding the aqueous phase to the oil phase while mixing in a conventional mixer at room temperature.

### EXAMPLES 1-3 - INCREASING THE SURFACTANT CONCENTRATION IN THE FORMUALTION

The following compositions were mixed by the above process.

| | | |
|---|---|---|
| Example 1 | Laurylmethiconecopolyol | 2% |
| | HMDS | 19.4% |
| | Silicone Gum | 0.6% |
| | Water | 78% |
| Example 2 | Laurylmethicone copolyol | 5% |
| | HMDS | 19.4% |
| | Silicone Gum | 0.6% |
| | Water | 75% |
| Example 3 | Laurylmethicone copolyol | 10% |
| | HMDS | 19.4% |
| | Silicone Gum | 0.6% |
| | Water | 70% |

Each of the above formulations were applied on the skin by rubbing with fingers. While applying, it was noted that initially the viscosity of the emulsions increased. The viscosity increase of Example 3 was greater than the viscosity increase of Example 2 which, in turn, was greater than the viscosity increase of Example 1. After a period of a few seconds of rubbing, each of the emulsions inverted resulting in a silicone coating on the skin with drops of water on top of the silicone coating.

These Examples show that increasing the amount of surfactant in the emulsion results in a greater viscosity increase during initial application while resulting in the same silicone films.

### EXAMPLES 4-5 - INCREASING THE AMOUNT OF SILICONE GUM IN THE FORMULATION

The following compositions were mixed by the above process.

| | | |
|---|---|---|
| Example 4 | Laurylmethicone copolyol | 2% |
| | HMDS | 24.25% |
| | Silicone Gum | 0.75% |
| | Water | 73% |
| Example 5 | Laurylmethicone copolyol | 2% |
| | HMDS | 22.5% |
| | Silicone Gum | 2.5% |
| | Water | 73% |

The above formulations were applied to the skin by rubbing with fingers. While applying, it was noted that the viscosity of the emulsions increased. The viscosity increase of Example 5 was greater than the viscosity increase of Example 4. After a period of about a few seconds, each of the emulsions inverted resulting in a silicone coating with drops of water on its surface.

These Examples show that increasing the amount of silicone gum in the emulsion results in a greater viscosity increase during initial application. In addition, the silicone film formed in Example 5 was thicker and more substantive than that formed in Example 4 because of the larger percentage of silicone gum in the formulation.

### EXAMPLES 6-8 - INCREASING THE AMOUNT OF SILICONE GUM AND VOLATILE SILICONE FLUID IN THE FORMULATION

The following compositions were mixed by the above process.

| | | |
|---|---|---|
| Example 6 | Laurylmethicone copolyol | 10% |
| | HMDS | 29.1% |
| | Silicone Gum | 0.9% |
| | Water | 60% |
| Example 7 | Laurylmethicone copolyol | 10% |
| | HMDS | 33.95% |
| | Silicone Gum | 1.05% |
| | Water | 55% |
| Example 8 | Laurylmethicone copolyol | 10% |
| | HMDS | 38.8% |
| | Silicone Gum | 1.2% |
| | Water | 50% |

Each of the above formulations were applied to skin by rubbing with fingers. While applying, it was noted that the viscosity of emulsions increased. The viscosity increase of Example 8 was greater than the viscosity increase of Example 7 which in turn was greater than the viscosity increase of Example 6. After a period of a few seconds, each of the emulsions inverted resulting in a silicone coating with drops of water on the surface.

This Examples show that increasing the amount of silicone gum and volatile silicone fluid in the emulsion results in a greater viscosity increase during initial application. In addition, the silicone film formed in Example 8 was thicker and more substantive than that formed in Example 7 and that of Examples 7 was thicker and more substantive than that of Example 6 because of the larger percentage of silicone gum in the formulation.

### EXAMPLES 9-10 - INCREASING VOLATILITY OF THE VOLATILE SILICONE FLUID

The following compositions were mixed by the above process.

| | | |
|---|---|---|
| Example 9 | Laurylmethicone copolyol | 2% |
| | HMDS | 20% |
| | Water | 78% |
| Example 10 | Laurylmethicone copolyol | 2% |
| | 1cSt | 20% |
| | Water | 78% |

The above formulations were applied to skin by rubbing with fingers. While applying, it was noted that the viscosity of the emulsions increased. The viscosity increase of Example 9 was greater than the viscosity increase of Example 10. After a period of a few seconds, each of the emulsions inverted resulting in a silicone coating with drops of water on the surface.

These Examples show that increasing the volatility of the volatile silicone fluid in the emulsion results in a higher viscosity increase during initial application.

### EXAMPLES 11-15 - INCREASING AMOUNT OF WATER IN THE EMULSION

The following compositions were mixed by the above process.

| | | |
|---|---|---|
| Example 11 | Laurylmethicone copolyol | 2% |
| | HMDS | 10% |
| | Dimethiconol blend | 5% |
| | Water | 83% |
| Example 12 | Laurylmethicone copolyol | 2% |
| | HMDS | 20% |
| | Dimethiconol blend | 5% |
| | Water | 73% |
| Example13 | Laurylmethicone copolyol | 2% |
| | HMDS | 22.5% |
| | Silicone gum | 2.5% |
| | Water | 73% |
| Example14 | Laurylmethicone copolyol | 2% |
| | HMDS | 40.5% |
| | Silicone gum | 4.5% |
| | Water | 53% |
| Example 15 | Laurylmethicone copolyol | 2% |
| | HMDS | 58.5% |
| | Silicone gum | 6.5% |
| | Water | 33% |

Each of the above formulations, were applied to the skin by rubbing with fingers. While applying, it was noted that the viscosity of emulsions increased. After a period of a few seconds, each of the emulsions inverted resulting in a silicone coating with drops of water on the surface. It was noted that the amount of water expelled from Example 11 was greater than that of Example 12 and the amount of water expelled from Example 13 was greater than the amount expelled from Example 14 which, in turn, was greater than the amount expelled from Example 15.

This Example shows that increasing the amount of water in the emulsion results in a greater expulsion of water. The silicone films in Examples 11 and 12 are the same due to the same amount of dimethiconol in the formulation. The silicone film in Example 15 is thicker and more substantive than that of Examples 14 and that of Example 14 is thicker than that of Example 13 because of the larger percentage of silicone gum in the formulation.

### EXAMPLES 16-17 - INCREASING AMOUNT OF WAX IN THE EMULSION

The following compositions were mixed by the above process.

| | | |
|---|---|---|
| Example 16 | Laurylmethicone copolyol | 2% |
| | HMDS | 24.25% |
| | Silicone Gum | 0.75% |
| | Silicone Wax | 5% |
| | Ethanol | 5% |
| | Water | 63% |
| Example 17 (Comparative) | Laurylmethicone copolyol | 2% |
| | HMDS | 19.4% |
| | Silicone Gum | 0.6% |
| | Silicone Wax | 10% |
| | Ethanol | 10% |
| | Water | 58% |

The above formulations were applied to the skin by rubbing with fingers. While applying, it was noted that the viscosity of the emulsions increased. After a period of a few seconds, Example 16 inverted resulting in a silicone coating with drops of water on the surface while Example 17 never inverted as demonstrated by the lack of the slipping sensation due to the absence of the water expulsion.

These Examples show that increasing the amount of wax in the emulsion results in inhibition of inversion.

### EXAMPLES 18-21 - ADDITION OF PROPYLENE GLYCOL TO THE EMULSION

The following compositions were mixed by the above process.

| | | |
|---|---|---|
| Example 18 | Laurylmethicone copolyol | 2% |
| | HMDS | 18% |
| | Dimethiconol Blend | 5% |
| | Silicone Wax | 2% |
| | Ethanol | 10% |
| | Water | 63% |
| Example 19 | Laurylmethicone copolyol | 2% |
| | HMDS | 18% |
| | Dimethiconol Blend | 5% |
| | Silicone Wax | 2% |
| | Ethanol | 5% |
| | Propylene Glycol | 5% |
| | Water | 63% |
| Example 20 | Laurylmethicone copolyol | 2% |
| | HMDS | 24.25% |
| | Silicone gum | 0.75% |
| | Water | 73% |
| Example 21 | Laurylmethicone copolyol | 2% |
| | HMDS | 24.25% |
| | Silicone gum | 0.75% |
| | Propylene Glycol | 10% |
| | Water | 63% |

Each of the above formulations were applied to skin by rubbing with fingers. While applying, it was noted that the viscosity of the emulsions increased. After a period of a few seconds, Example 18 inverted resulting in a silicone coating with drops of water on the surface while Example 19 inverted but with fewer water droplets visible on its surface. Similarly, Example 20 readily inverted whereas Example 21 inverted with fewer water droplets visible on its surface due to the humectant effect provided by the propylene glycol.

These Examples show that adding a greasy excipient to the emulsion results in reduced inversion as demonstrated by the fewer water droplets visible.

### EXAMPLES 22-23 - USE OF SORBITAN MONOOLEATE AS THE SURFACTANT AND ADDITION OF PARAFFIN OIL TO THE EMULSION

The following compositions were mixed by the above process.

| | | |
|---|---|---|
| Example 22 | Sorbitan Monooleate | 2% |
| | HMDS | 19.4% |
| | Silicone gum | 0.6% |
| | Water | 78% |
| Example 23 (Comparative) | Sorbitan Monooleate | 2% |
| | HMDS | 9.7% |
| | Silicone gum | 0.3% |
| | Paraffin oil | 10% |
| | Water | 78% |

The above formulations were applied to skin by rubbing with fingers. While applying, it was noted that the viscosity of emulsions increased. After a period of a few seconds, Example 22 inverted resulting in a silicone coating with drops of water on the surface while Example 23 never inverted.

These Examples show that an emulsion of the invention can be made with an organic surfactant. Similarly, it shows that adding a greasy excipient to the emulsion inhibits inversion.

### EXAMPLES 24-25 - ADDITION OF DIMETICONE TO THE EMULSION

The following compositions were mixed by the above process.

| | | |
|---|---|---|
| Example 24 (comparative) | Laurylmethicone copolyol | 2% |
| | HMDS | 15% |
| | Dimeticone | 5% |
| | Water | 78% |
| Example 25 (Comparative) | Laurylmethicone copolyol | 2% |
| | HMDS | 10% |
| | Linear pdms 20 cst | 5% |
| | Silicone wax | 2% |
| | Ethanol | 10% |
| | Water | 71% |

The above formulations were applied to skin by rubbing with fingers. Neither Example 24 or Example 25 inverted.

These Examples show that adding a greasy excipient to the emulsion inhibits inversion.

### EXAMPLES 26-29 - ADDITION OF ETHANOL TO THE EMULSION

The following compositions were mixed by the above process.

| | | |
|---|---|---|
| Example 26 | Laurylmethicone copolyol | 2% |
| | HMDS | 18% |
| | Dimethiconol Blend | 5% |
| | Silicone Wax | 2% |
| | Water | 73% |
| Example 27 | Laurylmethicone copolyol | 2% |
| | HMDS | 18% |
| | Dimethiconol Blend | 5% |
| | Silicone Wax | 2% |
| | Ethanol | 10% |
| | Water | 63% |
| Example 28 | Laurylmethicone copolyol | 2% |
| | HMDS | 19.4% |
| | Silicone Gum | 0.6% |
| | Ethanol | 20% |
| | Water | 58% |
| Example 29 | Laurylmethicone copolyol | 2% |
| | HMDS | 19.4% |
| | Silicone Gum | 0.6% |
| | Ethanol | 30% |
| | Water | 48% |

Each of the above formulations were applied to skin by rubbing with fingers. While applying, it was noted that the viscosity of the emulsions increased. After a period of a few seconds, each of the emulsions inverted resulting in a silicone coating with drops of water on the surface. It was noted that the viscosity increase of Example 27 was less than that of Example 26 and the viscosity increase of Example 29 was less than that of Example 28.

Theses Examples show that increasing the amount of a low volatility pharmaceutical excipient in an emulsion results in a lower viscosity increase prior to the inversion of the emulsion. The resultant films of Examples 26 and 27 and Examples 28 and 29 were the same because of the silicone gum content.

## Claims

1. In a method of forming a silicone coating on a substrate comprising depositing a composition comprising a water in silicone oil emulsion onto a substrate and then applying shear stress to the composition to distribute the composition on the substrate, the improvement comprising the use of an emulsion comprising 1 to 10 wt. % of a surfactant, 9 to 65 wt % of a volatile silicone fluid and 28 to 90 wt % of an aqueous phase, wherein the emulsion is one which breaks when the shear stress is applied to deposit a silicone coating on the substrate.

2. The method according to claim 1 wherein the volatile silicone fluid is selected from the group consisting of hexamethylcyclotrisiloxane, octamethylcyclotetrasiloxane, decamethylcyclopentasiloxane, hexamethyldisiloxane, octamethyltrisiloxane, and decamethyltetrasiloxane.

3. The method according to any of the previous claims wherein the volatile silicone fluid hexamethyldisiloxane.

4. The method according to any of the previous claims wherein the surfactant is an organic surfactant.

5. The method according to any of the previous claims wherein the surfactant is a polydiorganosiloxane-polyoxyalkylene block copolymer.

6. The method according to any of the previous claims wherein the emulsion also contains a silicone gum.

7. The method according to any of the previous claims wherein the emulsion also contains a silicone wax.

8. The method according to any of the previous claims wherein the emulsion also contains water soluble active ingredients in the aqueous phase selected from the group consisting of cosmetically active ingredients, pharmaceutically active ingredients and ingredients used in household and personal care.

9. The method according to any of the previous claims wherein the emulsion also contains oil soluble active ingredients selected from the group consisting of cosmetically active ingredients, pharmaceutically active ingredients and ingredients used in household and personal care.

10. The method according to any of the previous claims wherein the substrate is selected from hair and skin.

11. A method of forming a silicone coating on a substrate comprising depositing a composition comprising a water in silicone oil emulsion onto a substrate, wherein the composition comprises 1 to 10 wt. % of a surfactant, 9 to 65 wt % of a volatile silicone fluid and 28 to 90 wt % of an aqueous phase and wherein the emulsion is one which breaks when the shear stress is applied, and applying shear stress to the composition to distribute the composition on the substrate and thereby deposit a silicone coating on the substrate.

12. The method of claim 11 wherein the substrate is selected from hair and skin.

13. A composition comprising a water in silicone oil comprising
1 to 10 wt % of a surfactant;
9 to 65 wt % of a volatile silicone fluid;
28 to 90 wt % of an aqueous phase;
0 to 7 wt % of a silicone gum;
0 to 10 wt % of a non-volatile silicone fluid;
0 to 5 wt % of a silicone wax; and
0 to 30 wt % of active ingredients,
wherein the emulsion is one which breaks when shear stress is applied.
